# EUROPEAN PATENT SPECIFICATION

(11) **EP 2 170 327 B1**
(45) Date of publication and mention of the grant of the patent: **22.10.2014**
(21) Application number: 08761161.2
(22) Date of filing: 18.06.2008
(51) Int. Cl.: A61K 31/435, A61P 25/14

(54) **USE OF DOPAMINE STABILIZERS**
VERWENDUNG VON DOPAMIN-STABILISATOREN
UTILISATION DE STABILISATEURS DE LA DOPAMINE

(30) Priority: 18.06.2007 EP 07110478; 09.11.2007 EP 07120413
(43) Date of publication of application: 07.04.2010
(73) Proprietor: A.Carlsson Research AB, 413 19 Göteborg (SE)
(72) Inventor: CARLSSON, Arvid, S-413 19 Gothenburg (SE); TAMMINGA, Carol A., Dallas, Texas 75209 (US); CARLSSON, Maria L., S-413 23 Gothenburg (SE); RUNG, Johan, S-436 35 Askim (SE); NILSSON, Marie, S-422 49 Hisings Backa (SE)
(74) Representative: Stenbäck, Maria Elisabeth
(86) International application number: PCT/EP2008/057707
(87) International publication number: WO 2008/155357

(56) References cited:
- WO-A-01/46145
- WO-A-92/18475
- WO-A-02/056745
- US-A1- 2002 169 187
- RUNG ET AL: "The dopaminergic stabilizers (-)-OSU6162 and ACR16 reverse (+)-MK-801 induced social withdrawal in rats" PROG. IN NEURO-PSYCHOPHARMACOLOGY AND BIOLOGICAL PSYCHIATRY, vol. 29, 2005, - 2005 pages 833-839, XP004979072
- TEDROFF J ET AL: "Effects of the substituted (S)-3-phenylpiperidine (-)-OSU6162 on PET measurements in subhuman primates: Evidence for tone-dependent normalization of striatal dopaminergic activity" SYNAPSE 199804 US, vol. 28, no. 4, April 1998 (1998-04), pages 280-287, XP002497993 ISSN: 0887-4476
- TEDROFF J ET AL: "Long-lasting improvement following (-)-OSU6162 in a patient with Huntington's disease" NEUROLOGY, vol. 53, no. 7, 22 October 1999 (1999-10-22), pages 1605-1606, XP002497994 ISSN: 0028-3878
- NATESAN S ET AL: "The dopamine stabilizers (S)-(-)-(3-methanesulfonyl-phenyl)-1-propy l- piperidine [(-)-OSU6162] and 4-(3-methanesulfonylphenyl)-1-propyl-pip eridine (ACR16) show high in vivo D2 receptor occupancy, antipsychotic-like efficacy, and low potential for motor side effects in the rat" JOURNAL OF PHARMACOLOGY AND EXPERIMENTAL THERAPEUTICS 2006 US, vol. 318, no. 2, 2006, pages 810-818, XP002497995 ISSN: 0022-3565 1521-0103
- JOHANSSON ET AL: "Placebo controlled cross over study of the monoaminergic stabiliser (-)-OSU6162 in mental fatigue following stroke or traumatic brain injury", ACTA NEUROPSYCHIATRICA, 2012,

## Description

### Background

Dopamine stabilizers is a class of compounds which have the ability to reverse both hypo as well as hyperdopaminergia in vivo. This class may be exemplified by the phenylpiperidines (-)-OSU6162 and ACR16.

The compound (-)-OSU6162 (S-(-)-3-[3-methylsulfonylphenyl]-1-propylpiperidine) belongs to a group of phenylpiperidines developed in the Department of Pharmacology, University of Gothenburg, Sweden, in collaboration with The Upjohn Company, Kalamazoo, Michigan, USA (Sonesson et al. 1994). When characterized in animal in-vivo models this group of agents showed a profile similar to the partial dopamine D₂/D₃ receptor agonist (-)-3-PPP (S-(-)-3-[3-hydroxyphenyl]-1-propylpiperidine). This was especially true in regard to behaviour, in that not only inhibitory but also weakly stimulatory actions could be demonstrated, depending on the baseline activity level. Clearly the tendency to immobility ("catalepsy") even after high doses is weak (Natesan et al 2006), and extrapyramidal side effects did not occur in clinical studies demonstrating an antipsychotic action (Gefvert et al 2000, L. Lindström, unpublished data, Carlsson and Carlsson 2006). In these respects these agents are similar to (-)-3-PPP. Moreover, they show albeit moderate affinity for dopamine D₂ receptors, when studied in vitro, and their binding to these receptors is clearly documented in animal ex-vivo experiments. However, (-)-OSU6162 and its congeners did not show any signs of intrinsic activity on dopamine receptors in in-vivo or ex-vivo models (Sonesson et al 1994). These compounds, like (-)-3-PPP, are phenylpiperidines, but they differ from this agent in having electron-drawing substituents in 3-position on the phenyl ring rather than the OH group of (-)-3-PPP. The OH group has been assumed to be important for the partial and nearly full dopamine D₂/D₃-receptor agonist properties of (-)-3-PPP and (+)-3PPP, respectively.

In the absence of partial agonism the somewhat similar behavioural profile of (-)-OSU6162 and its congeners to (-)-3-PPP calls for an explanation. So far, attempts to clarify this issue have been based on the assumption of heterogeneity of dopamine D2 receptors (see Carlsson et al 2004, Carlsson and Carlsson 2006).

The substance (-)-OSU6162, or S-(-)-3-[3-methylsulfonylphenyl]-1-propylpiperidine), has so far been used in clinical dosages of at least 25 mg per day, and often higher. One major disadvantage is that it has been found that such high dosages may be connected to QTc values higher than normal, cardiac arrhythmia, or heart rhythm abnormalities, such as torsades de pointes.

### Summary of the invention

The present invention is based on the finding of the stimulating and inhibitory effects of the dopamine "stabilizer" (-)-OSU6162 on dopamine D₂ receptor function in vitro. The present invention is defined in the features of the invention at the end of the description and in the appended claims.

### Brief description of the drawings

Figure 1. Incorporation of GTPgammaS³⁵ in CHO-hD₂ₗ membranes in the presence of various concentrations of dopamine and (-)-OSU 6162. Overview of the entire experiment. For statistics, see legends to Figures 2 and 3.
Figure 2. Incorporation of GTPgammaS³⁵ in CHO-hD₂ₗ membranes following exposure to various concentrations of (-)-OSU 6162 in the absence of dopamine.
   Data are means and SD, expressed as cpm (zero values N=6, other groups N=3). ANOVA shows significant differences among treatment groups (F_{(4,12)}=4.76, P=0.0156). Zero values differ from 100 (P=0.0361), 1,000 (P=0.0067), and 10,000 nM (P=0.0032) concentrations of (-)-OSU 6162 . Differences between other groups are not significant.

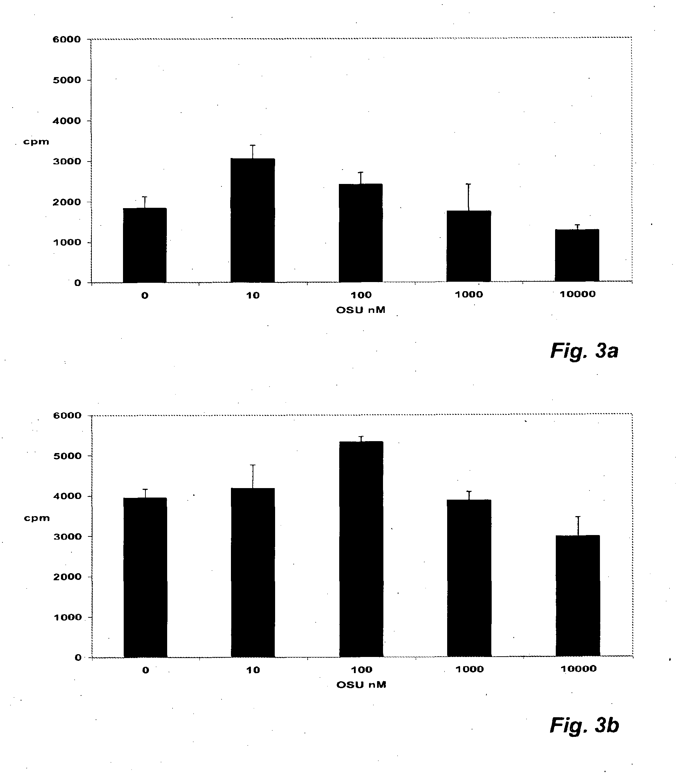

Figure 3. Incorporation of GTPgammaS³⁵ in CHO-hD₂ₗ membranes in the presence of two concentrations of dopamine (10 nM, Fig 3a, and 100 nM, Fig 3b) and various concentrations of (-)-OSU 6162.
   Data are means and SD of triplicates. They were obtained following subtraction from blank values, recorded in the absence of dopamine and (-)-OSU 6162. The blanks were 8,291±281 cpm (means±SD, n=6).
   For either dopamine concentration (10 nM, in Fig 3a; 100 nM, in Fig 3b) ANOVA showed significant differences among the treatment groups, F_{(4,10)}=4.36 and 16.05, respectively. In the 10 nM dopamine concentration series 10 nM (-)-OSU 6162 differed from zero (P=0.0246) and 10 nM (-)-OSU6162 differed from 1000 nM (p=0.0176 and 10000 nM (p=0.0033) (Fig 3a). In the 100 nM dopamine concentration series 100 nM (-)-OSU 6162 yielded values significantly greater than all the 4 other treatment groups (P=<0.003 in each case) (Fig. 3b), confirming a biphasic concentration-response curve.

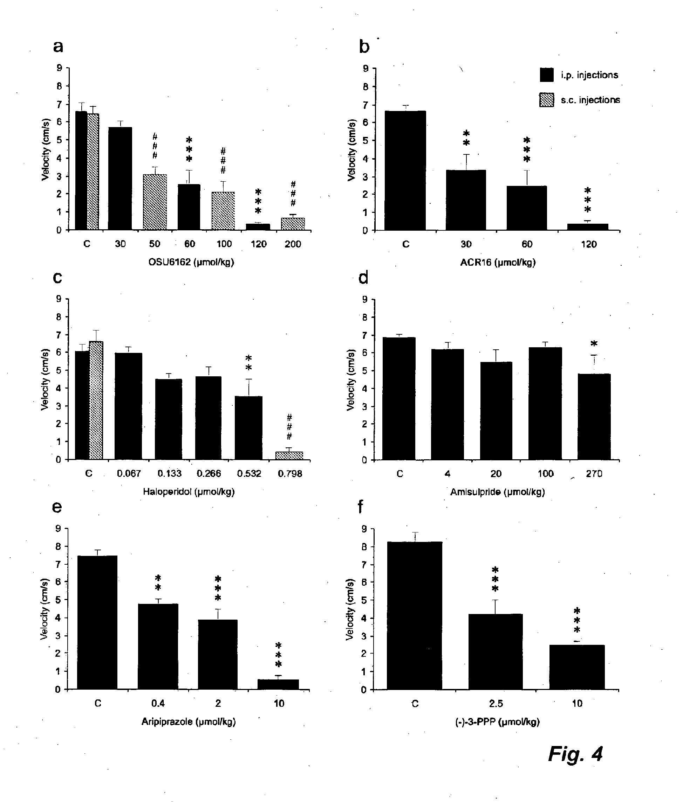

Figure 4. Effects of (a) OSU6162 (i.p., N=5; s.c. N=5), (b) ACR16 (i.p., N=5), (c) haloperidol (i.p., N=5-21; s.c., N=4), (d) amisulpride (i.p., N=4-9), (e) aripiprazole (i.p., N=4-5) and (f) (-)-3-PPP (s.c., N=5-6) on motor activity of active rats. Activity was measured as velocity in the video tracking setting. Drugs were administered 30 minutes prior to the 30 minute tracking period. Statistical comparisons were made versus control with univariate general linear model, followed by Dunnet's post hoc test. * p<0.05; ** p<0.01; *** p<0.001 (i.p. injections). ### p<0.001 (s.c. injections).

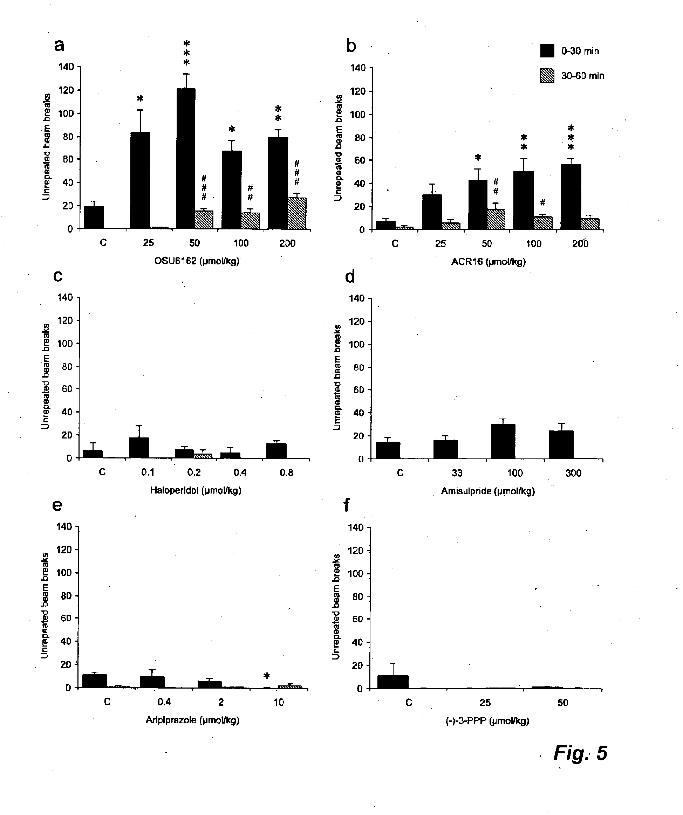

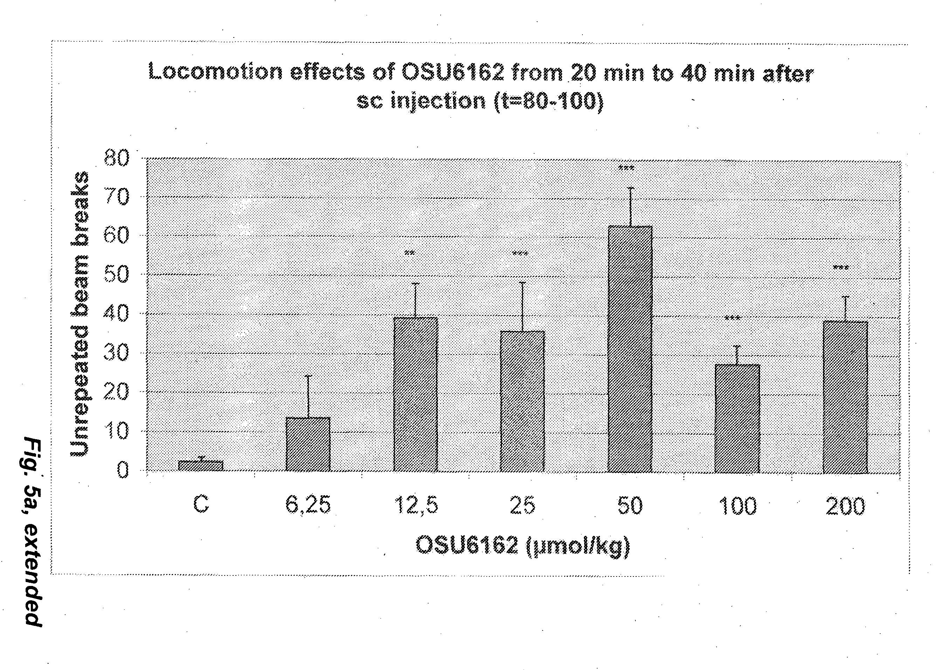

Figure 5. Effects of test drugs on motor activity of habituated rats in activity boxes during t=0-30 minutes and t=30-60 minutes after s.c. injection. (a) OSU6162 (N=7-10), (b) ACR16 (N=9-10), (c) haloperidol (N=4-5), (d) amisulpride (N=6-14), (e) aripiprazole (N=5-6), (f) (-)-3-PPP (N=4-5). Activity was measured as accumulated unrepeated beam breaks. Rats were allowed to habituate for 65 minutes and then injected with test drugs. This was followed by 60 minutes of registration. Statistically significant effects were established with Kruskal-Wallis one-way analysis of variance by ranks, and comparisons versus control were performed as described by Siegel and Castellan (1988). * p<0.05; ** p<0.01; *** p<0.001 (t=0-30 min). ^{#} p<0.05, ^{##} p<0.01, ^{###} p<0.001 (t=30-60 min).

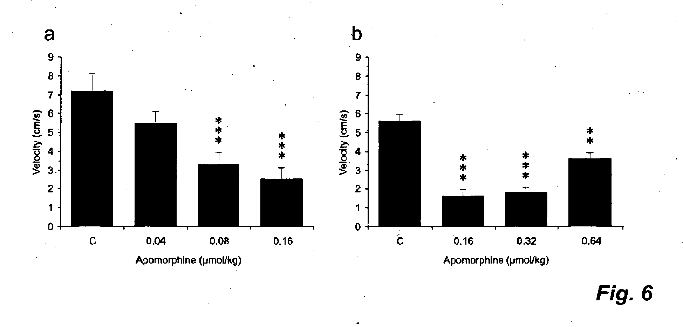

Figure 6. Effects of low doses of apomorphine (s.c.) on motor activity of active rats in the dose ranges (a) 0.04-0.16 µmol/kg (N=5) and (b) 0.16-0.64 µmol/kg (N=5). Motor activity was measured as velocity in the video tracking setting. Apomorphine was administered 5 minutes prior to registration and the rats were studied for 30 minutes. Statistical comparisons were made versus control with univariate general linear model, followed by Dunnet's post hoc test. ** p<0.01; *** p<0.001.

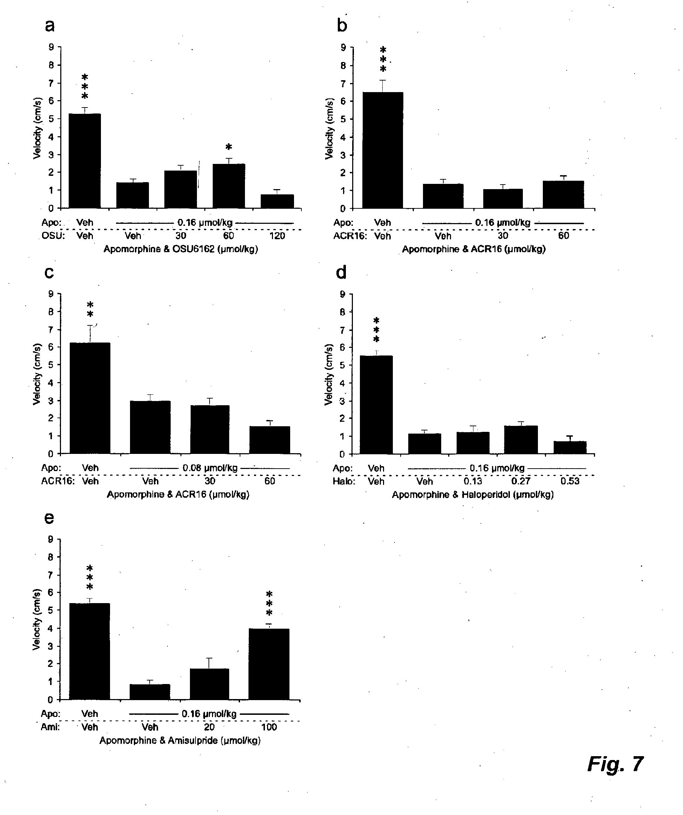

Figure 7. Effects of (a) OSU6162 (i.p., N=5-15), (b) ACR16 (i.p., N=10), (c) ACR16 (i.p., N=5), (d) haloperidol (i.p., N=5-15) and (e) amisulpride (i.p., N=5) on apomorphine induced hypomotility in rats. Apomorphine was administered s.c. 5 minutes prior to registration at the doses 0.16 µmol/kg (a-b, d-e) or 0.08 µmol/kg (c). Test drugs were administered 30 minutes prior to registration. Motor activity was measured for 30 minutes as velocity in the video tracking setting. Statistical comparisons were made versus the apomorphine group with univariate general linear model, followed by Dunnet's post hoc test. * p<0.05; * p<0.01; *** p<0.001.

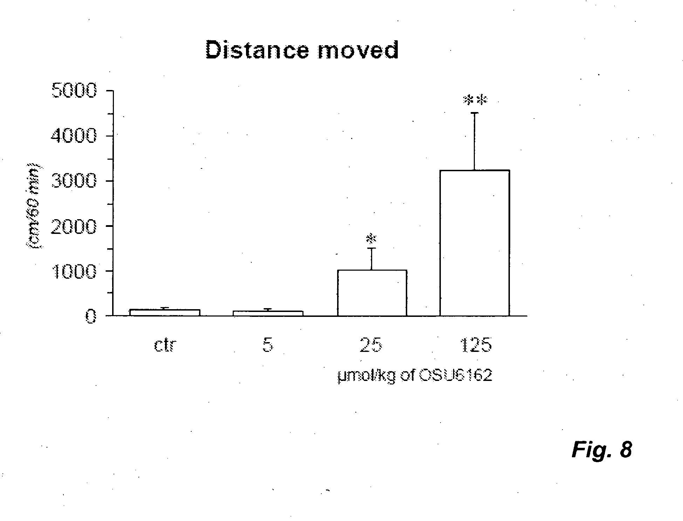

Figure 8. Effect of OSU6162 in a mouse model of Parkinsonism. Animals were pretreated with reserpine (10 mg/kg i.p.) and alpha-methyl-para-tyrosine (500 mg/kg i.p.) 18 and 4 hours before the experiment, respectively, and were then treated with various i.p. doses of OSU6162, expressed in µmol/kg, or saline ("ctr"). Immediately following injection the animals were placed in individual cages and video-taped from above for 60 min. Shown are the distances moved, means and S.E.M. N=5-6 per treatment group. Statistics: * P<0.05, **p<0.01, as compared with the controls.

### Detailed description of the invention

As already explained above, attempts to clarify the somewhat similar behavioural profile of (-)-OSU6162 and its congeners to (-)-3-PPP in the absence of partial agonism have so far been based on the assumption of heterogeneity of dopamine D₂ receptors. The inventors of the present invention have now found an additional, but not necessarily alternative, explanation. This is further explained below, i.a. by the in-vitro experiments on cloned dopamine D₂ receptors.

The inventors have found that low concentrations of a dopamine stabilizing compound such as (-)-OSU6162 enhances the stimulating action of dopamine, suggesting a weak partial agonism. However, this enhancing effect is reversed by higher concentrations of the dopamine stabilizing compound such (-)-OSU6162 in a complex biphasic manner. The dopamine-enhancing action is proposed to be mediated by binding to an allosteric site with high affinity and the inhibitory component by a low-affinity binding to the orthosteric site of the dopamine receptor.

The present invention relates to the medicinal use of a dopamine stabilizing substance, also called dopaminergic stabilizer or dopamine stabilizer, in a low dose, such as 1-20 mg.

The compound of formula I is S-(-)-3-[3-methylsulfonylphenyl]-1-propylpiperidine).

The clinical implications of the invention are at least twofold.

First, the discovery that the dopamine stabilizers described in the invention, can bind with high affinity to a putative allosteric site on the dopamine D2 receptor, leading to a stimulation of dopamine function, opens up new possibilities for treatment of a variety of neurological and psychiatric disorders characterized by a hypofunction of the dopamine system. In such cases the dosage of the stabilizer should be kept low to match the approximately 50 nanomolar concentrations needed for an adequate response, as further shown below. Although it is not possible to translate in-vitro data precisely to in-vivo dosages, it is reasonable to assume that the doses used so far, which were intended to match the micromolar affinities of the stabilizers to the orthosteric site of the same receptor, are severalfold higher than those needed to stimulate dopamine function via binding to the allosteric site. The oral doses used so far have been in the order of 25 to 150 mg per day. The doses needed for a stimulation of the dopamine system by binding to the allosteric site might then be at least 10 times lower, that is 2.5 to 15 mg per day. A general appropriate oral dosage according to the invention is from 1 to 20 mg per day, given once daily or divided in two equal doses. Suitable subcutaneous or intramuscular doses are only slightly lower, whereas intravenous doses are approximately 10 times less than the oral dosage.

Examples of disorders treatable according to the invention are:
Parkinson's disease in early stages, before introducing L-dopa or directly acting dopamine receptor agonists, or together with low doses of the same. Mental fatigue, associated with high age, stroke, postencephalitic and posttraumatic conditions.

However, the present invention has important additional clinical implications. In addition to the above-mentioned disorders, which are characterized by a hypofunction of dopamine, a large number of disorders rather seem to be due to a lack of stability of complex neural circuits controlled by dopamine neurons. In such circuits a dopamine stabilizer appears to be able to restore the stability even in cases where the primary cause of instability is located outside the dopamine system. A typical example is Huntington's disease, which is primarily due to degeneration of gabaergic neurons in the basal ganglia. Nevertheless, as shown in clinical trials using both (-)-OSU6162 and ACR16, these stabilizers can improve both signs of overactivity, e g chorea, and underfunctions, e g cognitive failure and depression. Such a stabilizing action is assumed to be due to blockade of mutually antagonistic subpopulations of the dopamine D2 receptor, such as the autoreceptors and the postsynaptic heteroreceptor. It has been assumed that autoreceptor is more readily blocked by a dopamine stabilizer than the postsynaptic receptor, and there is some evidence for that. However, the present invention introduces a new player, that is the allosteric site, on which the stabilizers can act in much lower concentrations than in the case of the orthosteric sites to counterbalance the hypofunction caused by blockade of the postsynaptic heteroreceptor. Obviously this should lead to the existence of a therapeutic window, where the optimum dosage should lie between those needed for a functionally relevant degree of binding to the allosteric and orthosteric sites, respectively. The consequence should then be that the optimum dosage for stabilization should lie between those needed for binding to the two sites, and thus lower than those previously assumed, which were based only on the data obtained from studies of the orthosteric sites. Thus it does not seem unlikely that the doses tried so far have been to high in relation to the therapeutic window, and thus that the benefit of the treatment has been underestimated. Thus the invention leads to the conclusion that future clinical trials in disorders characterized by instability of neural circuits must avoid too high doses by taking into account the probable existence of a therapeutic window. A proposed average dose intended for stabilization could be 25 - 50 mg per day, given once or preferably divided in two equal doses.

Examples of disorders, where an instability of neural circuits appear to be of crucial importance and where dopamine stabilizers in adequate dosage would be expected to be beneficial, are:
Schizophrenia, with positive or negative/cognitive deficit symptoms, or both. Other psychoses and paranoid conditions. Manic-depressive disorder. Huntington's disease (both chorea and mental symptoms, e g cognitive deficits). Tics, Tourette's disease, hiccup. Dyskinesias induced by L-dopa and other dopamine-receptor agonists. Tardive dyskinesias, induced by long-term treatment with dopamine receptor antagonists. Drug abuse and addiction (central stimulants, central depressants, alcohol, cannabis, opiates, nicotine). Addiction to gambling, certain foodstuffs etc. Emotional disorders, aggressiveness, pathological impulsiveness. Emotional disturbances induced by severe pain(treatment combined with analgesics). Anxiety disorders, panic disorders, generalized anxiety disorder, obsessive-compulsive disorder. Combined treatment with antipsychotic drugs, serving to alleviate their adverse effects (extrapyramidal, executive, cognitive, emotional).

One could consider a third category of disorders, which would be characterized by a pure hyperfunction of the dopamine system. However, the existence of such conditions may be questioned. Even if, for example, abuse of central stimulants initially leads to dopamine hyperfunction, addicts at the time of asking for medical help also suffer from instability and/or overt weakness of the dopamine system. As to spontaneous disorders, schizophrenia with pre-dominantly positive symptoms, indicating dopamine hyperfunction will also almost invariably be accompanied by signs of dopamine hypofunction (negative symptoms and cognitive deficits). The same thing is true of mania, and so forth.

The pharmaceutical composition according to the invention, used according to the invention or produced according to the invention may also comprise other substances, such as an inert vehicle, or pharmaceutical acceptable adjuvants, carriers, preservatives etc., which are well known to persons skilled in the art.

### Examples

The effect of (-)-OSU6162 on the incorporation of GTPgammaS³⁵ in the membranes of hD₂ₗ-transfected CHO cells was investigated. In the absence of dopamine the compound exerted a slight but significant stimulating action, suggesting a weak partial agonism. In the presence of dopamine, low concentrations (10 to 100 nM) enhanced the stimulating action of dopamine. This enhancing effect was reversed by higher concentrations of (-)-OSU6162 in a complex biphasic manner. The dopamine-enhancing action is proposed to be mediated by binding to an allosteric site with high affinity and the inhibitory component by a low-affinity binding to the orthosteric site of the dopamine receptor.

### Methods

Materials. (-)-OSU 6162 was obtained from Dr. Clas Sonesson (University of Gothenburg, Gothenburg, Sweden). Dopamine and GDP were purchased from Sigma Chemical Co. (St Louis MO). Unlabeled GTPgammaS was purchased from Roche (Indianapolis ,IN). All drugs were dissolved in deionized water and prepared daily. GTPgammaS³⁵ (1250 Ci/mmol) was purchased from NEN Life Science Products (Boston MA).

CHO-hD₂ₗ cells were grown to near confluence in T-150 flasks, and cells harvested, and membranes prepared as previously described (Lahti et al 1992). Membranes were stored at -80° C at a concentration of 1 mg protein/ml buffer.

Dopamine-stimulated GTPgammaS³⁵ incorporation was determined using the following procedure: in a buffer containing 20 mM HEPES, 5 mM Mg Cl2, 100 mM NaCl, at pH 7.5, membranes (60 µg/ml) were incubated with vehicle, drugs and 3 µM GDP for 30 min at 30° C, total volume was 1.1 ml. Then were added 30 microliter of GTPgammaS³⁵ (160 pM) and the incubation continued for another 30 min as before. The reaction was stopped by filtering the samples with a Brandel harvester on Whatmann GF/B filters. Filters were placed in liquid scintillation vials containing 10 ml of BCS scintillation fluid and counted the next day. All samples were run in triplicate.

### Results

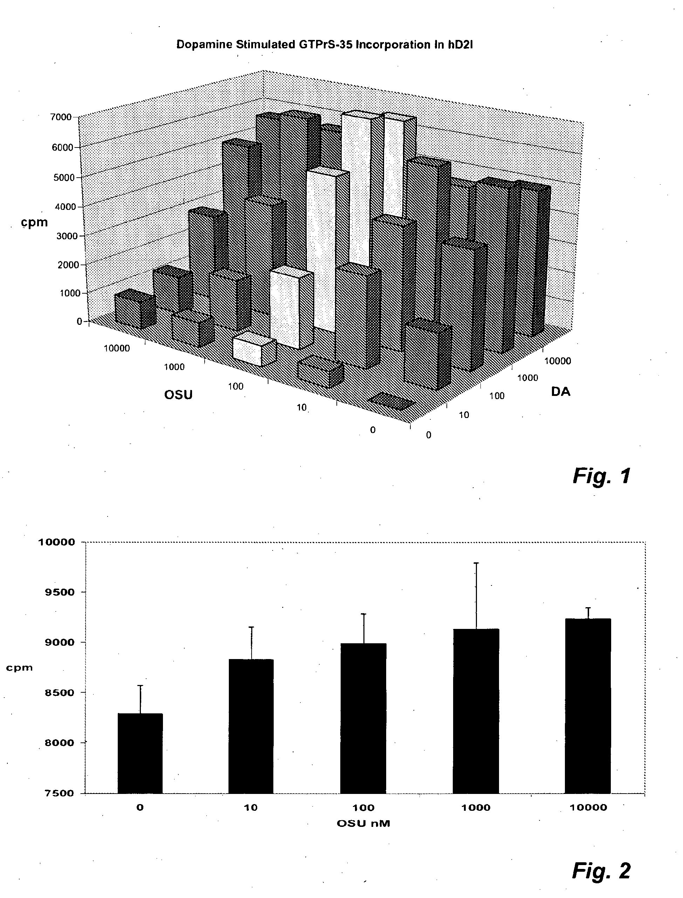

Figure 1 presents an overview of the effect of various concentrations of dopamine and (-)-OSU6162 on the incorporation of GTPgammaS³⁵. Following addition of dopamine alone a sigmoid-shaped curve is apparent, with a ceiling effect between 1,000 and 10,000 nM dopamine concentrations. A similar result was obtained in another experiment under the same experimental conditions.

In the absence of dopamine, 100 to 10,000 nM concentrations of (-)-OSU6162 increased the incorporation of GTPgammaS³⁵ by 8 to 11 percent (Figure 2, data extracted from Figure 1).

When dopamine and (-)-OSU6162 concentrations were varied simultaneously, the two agents showed a complex interaction on GTPgammaS³⁵ incorporation (Figure 3, data extracted from Figure 1). At low concentrations of dopamine (10 to 100 nM), low concentrations of (-)-OSU6162 (10 to 100 nM) caused an increased incorporation of GTPgammaS³⁵. However, this increase was no longer seen after higher concentrations of (-)-OSU6162 (1,000 to 10,000 nM). At higher dopamine concentrations (1,000 and 10,000 nM) variations in (-)-OSU6162 levels yielded similar, though flatter activity curves.

### Discussion

The data above demonstrate both stimulating and inhibitory effects of (-)-OSU6162 on the activity of dopamine D₂ receptors, expressed as GTPgammaS³⁵ incorporation. When given alone, (-)-OSU6162 was found to exert a weak stimulating effect on GTPgammaS³⁵ incorporation, suggesting that this agent is a partial agonist on dopamine D₂ receptors. In support of this hypothesis this compound is chemically closely related to 3PPP, whose enantiomers possess agonist properties on dopamine D₂/D₃ receptors. In addition (-)-OSU6162 has a behavioural profile fitting in with a weak partial agonism on dopamine D₂ receptors. Seeman and Guan (2006) also showed the mild agonist effect of (-)-OSU6162 on GTPgammaS³⁵ incorporation into cloned CHO cells with the D₂long dopamine receptor, but failed to demonstrate the complex interaction of varied concentrations of (-)- OSU6162 on the agonist effects. So far, however, direct in-vivo testing of (-)-OSU6162 has failed to demonstrate any dopamine receptor agonism (Natesan et al. 2006). The most likely explanation of the available data appears to be that (-)-OSU6162 is an albeit weak partial agonist on dopamine D₂ receptors. Probably such weak agonism is more easily detectable under in-vitro conditions, because a complete absence of dopamine can hardly be reached under in-vivo conditions.

When various concentrations of dopamine and (-)-OSU6162 were studied in combination, (-)-OSU6162 was found to have a biphasic effect on dopamine D₂ receptor activity. A stimulating action, enhancing the effect of dopamine, showed up after low concentrations of (-)-OSU6162, but this enhancing effect was reversed by higher concentrations, which thus seemed to antagonize the effect of dopamine.

The following interpretation would probably best fit in with previous knowledge about (-)-OSU6162. Both in-vitro binding and in-vivo data support the view that (-)-OSU6162 is an antagonist on dopamine D₂ receptors, albeit with a weak intrinsic activity, so far detectable only under in-vitro conditions. The antidopaminergic component induced by high concentrations of (-)-OSU6162 under the present conditions may be explained accordingly. This fits in with the fairly high Ki of the compound (in the micromolar region) in the previous in-vitro binding experiments (Sonesson et al. 1994).

The stimulation induced by low (-)-OSU6162 concentrations could then be due to binding to an allosteric site, leading to an enhanced action of dopamine, and possibly to the weak stimulation of the receptor observed even in the absence of dopamine. The binding of this compound to an allosteric site might lead to a conformational change of the receptor molecule, and this in turn could have a number of different consequences: a) an increased affinity to dopamine, b) an increased responsiveness to dopamine by some other mechanism, c) an increased constitutive activity of the receptor, or d) any combination between these possibilities. Alternative explanations may need to be considered, involving for example, binding to some adjacent protein which in one way or another might influence the dopamine receptor.

The complex mechanisms proposed above could perhaps be looked upon as a model for receptor stabilization: simultaneous occupancies by one and the same agent on allosteric and orthosteric sites, counterbalancing each other without necessarily disturbing the baseline receptor activity, should lead to a stabilizing action, because the amplitude of responses to endogenous dopamine release would be dampened, due to a reduced availability of binding sites. However, the extrapolation of data obtained in an artificial in-vitro system to in-vivo conditions must be regarded as tentative only.

The present experiments were performed on the long splice variant of the human D₂ receptor. It remains to be seen how the short variant will behave in this experimental model. There is a functional difference between the two variants at least in so far as auto- versus hetero-receptors are concerned: the former seem to belong predominantly to the short variant, whereas the latter are mixed (Khan et al 1998, Centonze et al 2002). The heteroreceptors are either synaptic or extrasynaptic. An attractive hypothesis would be that the short variant is predominantly extrasynaptic, regardless of whether it is an auto- or heteroreceptor, whereas the long variant would be primarily located to the synaptic cleft. The two variants would then be geared to different neurotransmitter concentrations. There is good evidence that the autoreceptors are more responsive to low transmitter concentrations than the heteroreceptor (Carlsson and Carlsson 2006). Moreover, postsynaptic dopamine receptors rendered extrasynaptic following degeneration of dopamine neurons become supersensitive. It would be interesting to compare the two splice variants in the present experimental model. In general, in-vitro binding experiments have so far shown them to behave equally, using a large number of antidopaminergic agents (Leysen et al 1993). However, one study has reported somewhat higher affinities of atypical antipsychotics to the short variant (Malmberg et al 1993).

The possible existence of an endogenous ligand for the proposed allosteric site could be a matter for speculation. Such a ligand could be released along with dopamine as a co-transmitter or from an adjacent cell. It could either enhance or dampen the function of dopamine and thus serve as an amplifier or stabilizer. The simultaneous existence of both amplifying and stabilizing allosteric ligands may also be considered.

### In-vivo data supporting the invention

### Summary

Dopaminergic stabilizers can be defined as drugs that stimulate or inhibit dopaminergic signalling depending on dopaminergic tone. (-)-OSU6162 and ACR16 appear to possess such a profile. They have been proposed to act as partial dopamine receptor agonists or as antagonists with preferential action on dopaminergic autoreceptors. Previous studies have shown either stimulation or inhibition of behaviour in response to OSU6162 and ACR16, which has been suggested to reflect their dual effects on dopaminergic signalling. The aims of the present work are to (1) examine the relation between behavioural response to these drugs and activity baseline, and (2) test the suggested mechanisms of action by means of close comparisons with the known partial D2-receptor agonists (-)-3-PPP and aripiprazole, and the D2 autoreceptor preferring antagonist amisulpride with respect to effects on behaviour. From the results of these experiments it can be concluded that: 1) The direction of the response to (-)-OSU6162 and ACR16 is dependent on activity baseline, which in turn, under physiological conditions, is determined primarily by test arena size of and degree of habituation to the environment. 2) The effects of (-)-OSU6162 and ACR16 cannot be explained on the basis of either partial dopamine receptor agonism or preferential dopamine autoreceptor antagonism. Nevertheless, the current data suggests at least two different D2-receptor-associated targets which mediate opposite effects on activity. This result fit in with a mechanism proposed from a recent in vitro study, according to which (-)-OSU6162 has a dual action on dopamine D2 receptors, a) an allosteric effect causing an enhanced response to dopamine, and b) the previously proposed orthosteric effect antagonizing the action of dopamine. The dosage reqiured for inducing the former effect is distinctly lower than that required for the latter..

### Introduction

Dopaminergic stabilizers constitute a novel principle for treating schizophrenia and other disorders involving dopaminergic circuits of the brain. Conceptually, these drugs act normalizing on dopaminergic signalling depending on dopaminergic tone. In case of an elevated dopamine function, these drugs cause dopaminergic inhibition. In case of a low dopaminergic tone, they enhance dopaminergic signalling. Thus, these drugs constitute potential treatments for a variety of conditions involving dopaminergic pathways. Focusing on schizophrenia, an appropriate degree of dopaminergic inhibition may alleviate positive symptoms without inducing the extrapyramidal and mental side effects associated with excessive D2-receptor blockade. Any negative symptoms caused by low dopaminergic tone could also improve, owing to the stimulating effect of these drugs.

One type of dopaminergic stabilizers are the partial D2-receptor agonists and include compounds like aripiprazole (Jordan et al., 2002a; Jordan et al., 2002b) and (-)-3-PPP (Carlsson, 1983). A partial agonist is per definition stabilizing on transmission at the level of the receptor, acting towards a degree of activation lower than that of the endogenous agonist. It will stabilize the average receptor activity towards a level that reflects its intrinsic activity, i.e. the relative preference for the active and inactive states of the receptor (see Buxton, 2005). Aripiprazole is among the partial D2-receptor agonists with the lowest known intrinsic activity (Jordan et al., 2007; Jordan et al., 2006; Tadori et al., 2005). Among the established partial D2-receptor agonists, only aripiprazole is on the market and is used in the treatment of psychosis and mania.

Another type of dopaminergic stabilizers appear to be pure D2 receptor antagonists and include the structurally related molecules (-)-OSU6162 (OSU6162) (compound no. 16, Sonesson et al., 1994) and ACR16 (Pontén et al., 2002; Waters et al., 2002). OSU6162 and ACR16 have been suggested to be D2 receptor antagonists with preferential action on autoreceptors (Carlsson et al., 2004)(see Carlsson et al., 2004). OSU6162, however, was recently claimed to be a partial dopamine receptor agonist with minor intrinsic activity (Seeman and Guan, 2006). OSU6162 and ACR16 have been investigated in early clinical studies and have been found therapeutically active in schizophrenia, Parkinson's disease with L-dopa induced dyskinesias, and in Huntington's disease (Gefvert et al., 2000; Lundberg et al., 2002; Tedroff et al., 1999, unpublished data; Information from Neurosearch A/S, Denmark, published in an International Offering Circular September 22, 2006). In this context the atypical antipsychotic amisulpride should be mentioned; it has been characterized as a preferential dopaminergic autoreceptor antagonist (Perrault et al., 1997; Schoemaker et al., 1997) but is usually not labelled a dopaminergic stabilizer.

Previously, in a test for social interactions, we observed inhibition of motor activity in response to OSU6162 and ACR16 in MK-801 treated rats but also in previously untreated rats (Rung et al., 2005a). The latter result contrasts with other studies, in which OSU6162 and ACR16 caused little or no activation in drug naïve rats placed in a new environment, or a clear-cut activation in animals habituated to the same environment (Natesan et al., 2006; Sonesson et al., 1994). We hypothesize that the main cause for these different outcomes is a difference in baseline activity. Behavioural inhibition was induced in rats with a high activity level; the high activity had been induced either by a new environment the rats found stimulating (Rung et al, 2005) or by treatment with dopaminergic enhancers, whereas activation was seen in inactive rats, previously habituated to a poorly stimulating environment (Sonesson et al, 1994; Natesan et al, 2006). Presumably, environmentally induced variations in motor activity level and in dopaminergic tone are closely interrelated. OSU6162 and ACR16 have been shown to reverse amphetamine-induced hyperactivity in rats (Natesan et al., 2006), indicating dopaminergic inhibition in a hyperdopaminergic state.

The aims of the present study were to (1) examine the influence of activity baseline on the behavioural response to OSU6162 and ACR16 and (2) test the previously presented hypotheses regarding the mode of action of these drugs by close comparisons with the well characterized compounds aripiprazole, (-)-3PPP and amisulpride in different behavioural settings.

### Materials and Methods

### Animals

Male Sprague-Dawley rats (Scanbur BK AB, Sollentuna, Sweden), weighing 255-340 g, were used in these experiments. Prior to testing, the rats were housed for approximately 1 week with free access to food and water, in groups of four or five in Macrolon type III cages in the animal facility of the Sahlgrenska Academy at Göteborg University. The experiments were approved by the animal ethics committee in Göteborg.

### Drugs

(-)-OSU6162 hydrochloride (MW=317.9), under the synonym PNU-96391A, was a gift from Pfizer Inc. (Groton, Connecticut, USA). Amisulpride (MW=369.5) was a gift from Sanofi-Aventis (Bagneux, France). ACR16 tartrate (MW=431.5) and aripiprazole (MW=448.4) were provided by Lilly Research Laboratories (Indianapolis, U.S.A). R-(-)-Apomorphine hydrochloride hemihydrate (MW=312.8), haloperidol (MW=375.9) and (-)-3-PPP hydrochloride (MW=255.8) were purchased from Sigma-Aldrich Sweden AB (Stockholm, Sweden).

OSU6162, ACR16, apomorphine and (-)-3-PPP were dissolved in 0.9 % saline. A few grains of ascorbic acid were added to apomorphine solutions to prevent oxidation of the drug. Haloperidol and amisulpride were dissolved in a minimum amount of acetic acid and diluted with 5.5% glucose. The solutions were adjusted to pH 5-7 with sodium bicarbonate. Aripiprazole was dissolved in a minimum amount of heated acetic acid, diluted in warm 5.5% glucose and adjusted to pH 4-5. All drugs were administered intraperitoneally (i.p.) or subcutaneously (s.c.) in volumes of 5 ml/kg. Control treatments consisted of the appropriate vehicle administered in accordance with the drug treatment at issue.

### Video tracking

This method was modified from a setting used previously for social interaction experiments (Rung et al., 2005b). The rats were housed in reverse daylight cycle and all handling of the animals was performed in dim light. Apomorphine was administered s.c. 5 minutes prior to testing, while all other drugs were injected i.p. or s.c. 30 minutes before testing. Single rats were introduced into rectangular arenas (l/w/h: 150×100×40 cm) illuminated indirectly by one infrared lamp (Neocom, South Korea). The rats' movements were recorded to digital (MPEG2) video files using an IR sensitive video camera (Panasonic WV-CPR480, lens: Panasonic LA-408C3) connected to a PC equipped with a MPEG-encoder (MVR1000^{SX}, Canopus Co.). The video files were then analyzed with the video tracking software EthoVision 3.1 Color Pro (Noldus Information Technology, Wageningen, The Netherlands) using a sample frequency of 12.5 samples per second. Behavioural variables were extracted from the tracks in MatLab (The MathWorks, Inc., USA) with functions developed in this laboratory. Mean velocity was used to measure motor activity. Prior to calculation of velocity the tracks were subjected to a running mean filter, i.e. each sample was replaced by the mean of fifteen consecutive samples.

### Motor activity in activity boxes

These experiments were designed to resemble those of Sonesson et al (1994). Rats were housed in a normal daylight cycle and testing was performed during daytime. Animals were introduced into sound attenuated, illuminated activity boxes (l/w/h: 40×40×20 cm) and were allowed to habituate in the new environment for 65 minutes. During the following 5 minutes test drugs were injected s.c., after which the rats were returned to the boxes for another 60 minutes. Five by five rows of photocell beams at floor level allowed a computer based-system to register horizontal activity. Motor activity is presented as accumulated number of unrepeated beam breaks, i.e. several consecutive breakings of one beam is counted as one beam break. The software was set to register behaviour from the rat's first introduction into the arena until it was finally removed from the box, i.e. for 130 minutes. Activity is presented separately for t=0-30 min and t=30-60 min after injection.

### Statistics

In several cases data from two or more experiments have been pooled. Suspected outliers were evaluated one at the time with Grubbs' test for outliers (De Muth, 1999; Grubbs, 1969). Confirmed outlying values (N=0-3 per experiment) were excluded from statistical analyses and graphical presentations. Data from the video tracking setting was analyzed with univariate general linear model (GLM). Following GLM analysis, comparisons versus the appropriate control groups were performed with Dunnet's post hoc test. GLM analyses showed no statistically significant inter-experiment differences in pooled data with respect to drug effects on motor activity. "Experiment" was therefore not included as a factor in the GLM analyses. Data obtained from activity boxes was analyzed with Kruskal-Wallis one-way analysis of variance by ranks. Comparisons were made versus control as described by Siegel and Castellan (1988). All statistical tests were two sided and p<0.05 was considered statistically significant.

### Results

### Effects on motor activity in active rats

In the video tracking setting, control rats had a high initial activity level, and activity decreased markedly, but did not level out, during the 30 minutes of behavioural testing.

Both OSU6162 and ACR16 (30-120 µmol/kg, i.p.) caused a dose-dependent and statistically significant reduction of motor activity (Figure 4a-b). When administered subcutaneously OSU6162 (50-200 µmol, s.c.) induced a similar dose dependent behavioural inhibition (Figure 4a). The classical neuroleptic haloperidol (0.067-0.53 µmol/kg) caused statistically significant inhibition at the highest dose (Figure 4c). However, rats receiving the highest dose of haloperidol may be divided into one group (N=4) not responding to the treatment and one group (N=6) with a marked reduction in motor activity. This indicates that this dose may be on the threshold between response and no response with respect to activity. When administered subcutaneously at a higher dose, haloperidol (0.8 µmol/kg, s.c.) inhibited motor activity to approximately 7 % of control level (Figure 4c). Amisulpride (4-270 µmol/kg, i.p.), an autoreceptor preferring D2/D3-receptor antagonist, inhibited motor activity slightly at the highest dose tested (Figure 4d). The partial D2-receptor agonists aripiprazole (0.4-10 µmol/kg, i.p.) (Figure 4e) and (-)-3-PPP (2.5 and 10 µmol/kg, s.c.) (Figure 4f) induced marked and dose-dependent inhibition on motor activity, that was statistically significant at all doses tested.

### Effects on motor activity in inactive rats

The rats were allowed to habituate in the boxes for 65 minutes before injections of test drugs. During the habituation period there was a steep decline in motor activity. After approximately 30 minutes, motor activity had reached a baseline that was close to zero. Following injection, the rats displayed a moderate but unmistakable motor activation. Control animals typically became stationary within 10 minutes after injections, and remained so until removed from the boxes.

All doses of OSU6162 (25-200 µmol/kg, s.c.) induced a marked locomotor activation during the first 30 minutes following drug injection. During the final 30 minutes of registration, the three highest doses significantly stimulated locomotion (Figure 5a). ACR16 (25-200 µmol/kg, s.c.) also caused an activation, although smaller than that of OSU6162. During the first 30 minutes after injection this effect increased dose-dependently. During the final 30 minutes of observation, the effect appeared to peak at the dose 50 µmol/kg (Figure 5b). Although OSU6162 and ACR16 clearly stimulated activity of habituated rats, the resulting activity level was low compared the activity before habituation. Neither haloperidol (0.1-0.8 µmol/kg) nor amisulpride (33-300 µmol/kg) had any significant effects on motor activity (Figure 5c-d). Aripiprazole (0.4-10 µmol/kg, s.c.) caused a dose-dependent inhibition during the first 30 minutes after injection, which was statistically significant at the highest dose, but did not affect locomotor activity during the final 30 minutes of registration (Figure 5e). (-)-3-PPP did not have any statistically significant effects on motor activity in either time interval (Figure 5f).

In a later experiment the data showed in Figure 5a were extended to include two lower doses of OSU6162, i e 6.25 and 12.5 µmol/kg, s.c.(see Figure 5a extended, showing data from all doses). A significant effect shows up already in a dose of 12.5 µmol/kg that is, a dose severalfold lower than that required for inducing behavioural inhibition (see Fig. 4a).

### Effects on apomorphine-induced hypoactivity

Apomorphine had a dose dependent inhibiting effect on locomotor activity at the doses 0.04-0.16 µmol/kg (s.c.), when tested in the video tracking setting (Figure 6a). When a higher dose-interval (0.16-64 µmol/kg) was tested it was apparent that motor activity began to return towards a level similar to that observed in controls (Figure 6b). Maximal inhibition of apomorphine occurred at the dose 0.16 µmol/kg, which also appears to be a standard dose for this type of experiments (e.g. Ståhle and Ungerstedt, 1986; Svensson et al., 1986).

Several drugs were tested regarding their effects on apomorphine (0.16 µmol/kg, s.c.) induced hypomotility. OSU6162 (30-120 µmol/kg, i.p.) caused a modest but apparently dose dependent reversal of apomorphine-induced hypomotility up to a dose of 60 µmol/kg. A higher dose (120 µmol/kg), however, seemed to cause a marked drop in motor activity (Figure 7a). ACR16 (30, 60 µmol/kg, i.p.) did not have any noticeable effect on apomorphine-induced hypoactivity (Figure 7b). When tested with a lower dose of apomorphine (0.08 µmol/kg, s.c.), the higher dose of ACR16 tended to inhibit activity further (Figure 7c). Haloperidol (0.13-0.53 µmol/kg, i.p.) had no statistically significant effects on motor activity when added to apomorphine (0.16 µmol/kg, s.c.) (Figure 7d). Amisulpride (20-100 µmol/kg, i.p.) effectively counteracted apomorphine-induced hypoactivity (Figure 7e). When tested at a higher dose (270 µmol/kg, s.c.), amisulpride induced a mere tendency towards reversal of apomorphine-induced hypoactivity (data not shown).

### Discussion

This study shows that the dopaminergic stabilizers OSU6162 and ACR16 inhibit motor activity in drug naïve rats with a high activity level but have the opposite effect in rats with a low activity level, i.e. there is a stabilization towards an intermediate level of behavioural activity. To our knowledge, this has not previously been shown within the same study.

Already in an earlier study we observed behavioural inhibition in response to OSU6162 and ACR16 in drug naïve rats (Rung et al., 2005a). Inhibition in response to these drugs has also been observed in rats treated with amphetamine and apomorphine (unpublished data, Dept. of Pharmacology, Göteborg University, summary graph published in Carlsson, 2001; Natesan et al., 2006). In previous studies on drug naïve rats these drugs were reported to influence behaviour differently depending on baseline activity. In animals activated after being introduced into a new environment the effect was slight and variable (Sonesson et al., 1994), whereas it was clearly stimulating in animals with low activity following habituation to the environment (Natesan et al., 2006; Sonesson et al., 1994). The latter response was also observed in the current investigation using a similar setting.

The question arises why an inhibitory effect was not seen in the earlier studies in drug naïve rats, apart from that by Rung et al (2005a). In this context many factors should be considered, e g area of the test arena, degree of habituation, drug pre-treatment time, drug administration route, day-light cycle and illumination. We have reached the conclusion that test arena size and degree of habituation to the environment are most important in determining the behavioural responses to OSU6162 and ACR16. The surface area of the arenas was almost an order of magnitude larger in experiments by Rung et al, including the present study. It is reasonable to assume that the larger arenas are more stimulating. In studies where OSU6162 and ACR16 fail to reduce activity, rats are studied in small activity boxes and injections are made immediately before the rats are introduced into the equipment. By observing the recordings from the habituation period in the current study, we find that the rats habituate almost fully to these small activity boxes within the first 15 minutes of registration. Thus, when the drugs are administered immediately before registration of behaviour, the rats will be partly habituated before the drugs have reached full effect. In a preliminary experiment ACR16 indeed caused marked reduction in motor activity in small activity boxes; in this experiment, we used a pre-treatment time of 30 minutes and the rats were studied during the dark hours, with the light inside the boxes turned off.

The administration routes were often not the same in the different procedures and studies. Therefore, we performed two additional experiments to study the importance of administration route. OSU6162 and haloperidol were selected to be administered s.c. in the video tracking setting. The outcome after s.c. administration corresponded well with the response to i.p. injections of the drugs. Thus, at least for these two compounds, administration route does not seem to be of major importance for the behavioural response.

As to the issues of illumination and daylight cycle, behavioural inhibition has been observed in ultraviolet (Rung et al., 2005b) or infrared light (current study) during the dark hours of the rats' diurnal rhythm. Based on how the light is perceived by humans, we presume that the ultraviolet and infrared light used in these studies is perceived by the rats as dim light and darkness respectively. In one preliminary experiment, rats studied during daytime in large illuminated arenas responded to OSU6162 with a behavioural inhibition similar to that observed in darkness. Preliminary data also showed that rats habituated to this environment did not respond with any change in activity following treatment with OSU6162.

In summary, our observations show that whether OSU6162 and ACR16 will have stimulatory or inhibitory effects on behaviour is dependent on the rats' activity and arousal level. This, in turn, is under physiological conditions determined primarily by (1) the size of the arena and (2) the degree of habituation to the environment. These two are strongly interconnected since the habituation rate is greatly affected by arena size. Administration route, light conditions and daylight cycle generally seem to be factors of minor importance. Our observation that an inhibitory influence of OSU6162 and ACR16 on behaviour can be demonstrated without pre-treatment with any stimulants, i.e. under physiological conditions, is of course important and further underscores the stabilizing behavioural profile of these agents.

The data reported in the present invention, where we have made close comparisons between OSU6162 and ACR16 on one hand and established dopamine partial agonists and a D2 autoreceptor selective antagonist on the other, permit certain conclusions to be drawn regarding the mechanisms underlying the stabilizing profile of the former drugs:

As mentioned in the Introduction, partial dopamine agonists can be characterized as stabilizers, because they can serve as mixed agonists/antagonists. A partial agonist is more likely to behave as an agonist when the endogenous ligand is of low abundance, while the antagonist properties show up more easily when the endogenous agonist concentrations are high. At the receptor level, a partial dopaminergic agonist affects dopaminergic transmission towards a level reflecting its intrinsic activity; this is influenced by the receptor responsiveness, which in turn is affected by the degree of previous long-term stimulation of the receptor (see Carlsson, 1983). Recently it was reported that OSU6162 has a minor agonist effect at D2-receptors *in vitro* (Lahti et al., 2007; Seeman and Guan, 2006). According to another study, ACR16 does not seem to have this effect (Tadori et al., 2007). In spite of their higher intrinsic activity at D2-receptors, neither aripiprazole nor (-)-3-PPP induced activation in inactive rats. Thus, the present data show convincingly that the behaviourally stimulating action of OSU6162 and ACR16 cannot be explained by partial dopamine receptor agonism in the classical sense. Further compelling evidence for the lack of even partial agonism by OSU6162 or ACR16 in the classical sense under in-vivo conditions has been summarized by Natesan et al. (2006; see page 816).

In the present study, rats with high activity level as a result of the introduction to a novel stimulating environment react to OSU6162 and ACR16 treatment with marked inhibition of behaviour. This is probably due to an elevated dopamine activity. In support of this assumption, it has been shown that, when rats are placed in a new environment, extracellular dopamine levels increase in the nucleus accumbens (Rebec, 1998). We are currently analysing brains from active and inactive rats for any biochemical dopamine-related differences. Preliminary data indicates that active rats, compared to inactive rats, have higher concentrations of homovanillic acid in the striatum, indicating a higher dopaminergic activity. Furthermore, OSU6162 and ACR16 have been shown to reverse the activating effect of amphetamine in rats (Natesan et al., 2006) and sub-human primates (Brandt-Christensen et al., 2006). It is reasonable to assume that the inhibitory effect seen in this study is due to a reversal of a physiologically induced increase in dopaminergic tone and due to blockade of postsynaptic receptors. Thus, it seems that OSU6162 and ACR16 act on at least two different targets with opposite effects on activity and dopaminergic signalling.

The behavioural activity level of animals can be influenced by dopamine receptors in both directions: stimulation of autoreceptors and heteroreceptors leads to behavioural inhibition and activation, respectively, and blockade of either of these receptors has the opposite effect. As mentioned, OSU6162, ACR16 and their congeners are for all practical purposes to be looked upon as dopamine D2/D3 receptor antagonists, because in the in vivo situation we can disregard the minimal intrinsic activities that may show up *in vitro* (Lahti et al., 2007; Seeman and Guan, 2006; Sonesson et al., 1994). We have previously considered the possibility that the ability of these agents to stimulate or inhibit behaviour could be the result of shifting the balance between the two opposing actions, namely blockade of autoreceptors and heteroreceptors respectively. In other words, their stimulating action could be due to a preferential autoreceptor antagonism.

For detecting autoreceptor preference of OSU6162 and ACR16 we tested these drugs for reversal of apomorphine-induced hypoactivity. We used the dose 0.16 µmol/kg (0.05 mg/kg) of apomorphine, since it was shown to be the most effective in inhibiting activity in this setting. OSU6162 induced a partial reversal of apomorphine-induced hypoactivity, which indicates a modest preference for D2 autoreceptors versus synaptic receptors. ACR16, however, had no such effect in this model. Since ACR16 has a lower affinity than OSU6162 for D2-receptors, it may be argued that this drug may not be capable of competing with apomorphine for the binding sites of D2 autoreceptors. Therefore, we also tested ACR16 along with a lower dose (0.08 µmol/kg) of apomorphine. Under these conditions ACR16 again did not counteract apomorphine-induced hypomotility but rather tended to further inhibit behaviour, an effect that was probably due to blockade of D2 heteroreceptors.

Previously amisulpride has been convincingly characterized as a preferential dopamine autoreceptor antagonist (Perrault et al., 1997). We thus considered it of great interest to compare the behavioural action of this agent closely with OSU6162 and ACR16. The outcome of this comparison was very clear. The preferential action of amisulpride on dopaminergic autoreceptors could be readily confirmed by demonstrating its reversal of apomorphine-induced hypomotility. Despite the superiority of amisulpride over OSU6162 and ACR16 in this autoreceptor preference model, it turned out to be unable to stimulate the behaviour of habituated animals. Thus one can exclude the kind of preferential autoreceptor antagonistic action caused by amisulpride as the sole or even a major mechanism underlying behavioural activation induced by OSU6162 and ACR16. This would be true of both D2 and D3 autoreceptors, because amisulpride seems to act equally strongly on these two receptor subtypes (Schoemaker et al., 1997).

The inability of amisulpride to inhibit behaviour in active rats over a large dose range, as shown in the present study (cf Perrault et al., 1997), is remarkable and may suggest a low ability of this compound to reach synaptic receptors. This would fit well with the observations that amisulpride shows poor penetration of biological membranes (Hartter et al., 2003). Amisulpride has previously been shown to reverse behavioural activation induced by amphetamine and apomorphine (Perrault et al., 1997), which indicates that heightened arousal in response to stimulating drugs is qualitatively different from that occurring under physiological conditions and may result from blockade of extrasynaptic receptors (see Carlsson et al, 2004). Since also the autoreceptors are extrasynaptic it would thus appear that amisulpride is a compound with selectivity for extrasynaptic receptors, possibly due to pharmacokinetic factors.

To summarize, our present results have shown that neither partial dopamine receptor agonism nor preferential dopamine autoreceptor antagonism can be the main mechanism underlying the unique behavioural profiles of OSU6162 and ACR16 and, hence, we had to search for a third mechanism:
In a recent study OSU6162 was found to possess both stimulating and inhibitory effects on D2-receptor activity in an *in vitro* assay measuring GTPγS incorporation in membranes from CHO-cells transfected with human D2l receptors. Low concentrations of OSU6162 potentiated the effect of dopamine while higher concentrations inhibited receptor activity, resulting in a biphasic concentration-response curve. In the absence of dopamine OSU6162 had a minor stimulating effect, suggesting that the compound has weak intrinsic activity. The interpretation of these data is that OSU6162 enhances D2-receptor activation via an allosteric site at the receptor whereas inhibition is attributed to blockade of the binding site for dopamine, i.e. the "orthosteric" site (Lahti et al., 2007, see above pp. 12-16). These mechanisms provide strong and welcome candidates for the two targets for OSU6162 and ACR16 suggested by the current data. The allosteric and orthosteric sites of the D2-receptor would then be responsible for behavioural activation and inhibition, respectively.

The phenylpiperidine derivatives OSU6162 and ACR16 with dopamine-stabilizing properties discussed in this paper have in general a higher affinity to dopamine D3 than D2 receptors, judging by in-vitro data (Sonesson et al., 1994). The question arises whether the inhibiting effects of these drugs could be due to a preferential binding to D3 receptors. At one time the latter subtype was believed to be behaviourally inhibitory and thus antagonistic to the former subtype (Waters et al., 1994). More recently, however, the weight of evidence indicates that both D2 and D3 heteroreceptors (that is, postsynaptic receptors) are behaviourally activating (see Sokoloff et al., 2003). When considering partial agonists, (-)-3-PPP has been found to be a partial agonist also on dopamine D3 receptors (Malmberg et al., 1998), and thus the argument put forward above against partial agonism underlying the behavioural activation by OSU6162 and it congeners would be equally valid also for D3 receptors. In the case of autoreceptor antagonism involving D3 receptors the same argument can be applied, because amisulpride has about the same affinity for D2 and D3 receptors, as observed *in vitro* (Schoemaker et al., 1997).

In this context it is interesting to mention the fairly strongly D3- versus D2-preferring compound U99194A, which led to the suggestion that postsynaptic D3 heteroreceptors had a behaviourally inhibitory function. This drug was shown to induce an unusually strong behavioural activation, but also to potentiate the stimulating effects of amphetamine and apomorphine on behaviour (Waters et al., 1994; Waters et al., 1993). These are effects that could be expected from a drug with a pronounced preference for the proposed allosteric site at the D2-receptor. In light of recent observations, and the results of the current investigation, the data presented in these papers would appear to fit in best with the hypothesis that this compound binds to both the allosteric and orthosteric sites of dopamine D2 receptors. This can be looked upon as an example of the necessity to revisit earlier pharmacological data in light of recent findings.

In conclusion, this study has shown that the dopaminergic stabilizers OSU6162 and ACR16 inhibit motor activity in drug naïve rats with a high activity level but have the opposite effect in rats with a low activity level. This suggests that these drugs stabilize dopaminergic signalling even under physiological conditions. The effects of (-)-OSU6162 and ACR16 cannot be explained on the basis of either partial dopamine receptor agonism or preferential dopamine autoreceptor antagonism. Nevertheless, our results suggest that (-)-OSU6162 and ACR16 act via at least two D2-receptor associated targets which have opposing actions on dopaminergic transmission. This would fit in with a mechanism proposed from the in vitro study referred to above, according to which (-)-OSU6162 has a dual action on dopamine D2 receptors, a) an allosteric effect causing an enhanced response to dopamine, and b) the previously proposed orthosteric effect antagonizing the action of dopamine.

The present study makes it possible, for the first time, to compare the dose requirements for the two mutually antagonistic actions of a dopamine stabilizer such as OSU6162. The dose required for inducing a stimulatory effect on behaviour was found to be distinclly lower than that required for inducing inhibition (compare Figure 4a with Figure 5a, extended).

### Additional data, using a mouse model of Parkinsonism.

In these experiments we used male NMRI mice weighting 25-30 g at the time of testing. The mice were pretreated with reserpine, 10 mg/kg i.p., 18 hours, and alpha-methyl-para-tyrosine HCl, 500 mg/kg i.p. 2 hours, respectively, before the experiment. This treatment has been found to induce virtually complete immobility and also to block the stimulating action of dopamine-releasing agents such as amphetamine.

The actual experiment was started by injecting either OSU6162 in doses of 5, 25 or 125 µmol/kg i.p.or placebo (a physiological saline solution). Each dosage group consisted of 5-6 mice. Immediately after injection the mice were placed in individual cages and video taped from above for 60 min. Various aspects of motility were analysed from the video tapes.

As shown in figure 8, the controls as well as the animals treated with the lowest dose of OSU6162 (5 µmol /kg), showed only minimal signs of motility. The two higher doses showed a significant, dose dependent increase in motility, with a pattern hardly distinguishable from normal.
Statistical calculations were performed with the non parametric Mann-Whitney U-test, and asterisks in the graph indicate significant differences compared to control group (*p<0.05; **p<0.01).

It is surprising that this impressive, potentially "therapeutic" action of OSU6162 in an animal Parkinson model has not previously been detected. Similar data from rat experiments have been published (Sonesson et al. 1994), and they have generally been interpreted as indicating lack of activity. However, a closer examination of these data, in light of the present observations, call for some caution regarding the interpretation. Obviously more studies are needed in order to reach definite conclusions regarding the anti-Parkinson activity of OSU6162 in rat models.

### Summary of in-vitro and in-vivo data supporting the invention

In the in-vitro experiments OSU6162, a prototypic dopamine stabilizer belonging to a class of phenylpiperidines, was found to exert a biphasic action on the activity of human D2l receptors, with a stimulating component in lower concentrations (10 to 100 nM) and an inhibitory component in higher concentrations.

In the in-vivo experiments on rats OSU6162 and ACR16 were found to stimulate the low activity of animals with low wakefulness, induced by habituation to an environment in small cages. This stimulation occurred already in low dosage, a finding reported for the first time in the present invention. On the other hand, these drugs exerted an inhibitory effect on animals with a high wakefulness, induced by the novelty of a stimulating environment. This inhibition required distinctly higher doses than the stimulation of habituated animals.

This pattern of behaviour was different from those found in the established partial dopamine-receptor agonists (-)-3PPP and aripiprazole, which proved unable to stimulate the behaviour of habituated animals. It also differed from the preferential dopamine autoreceptor antagonist amisulpride, which likewise proved unable to stimulate the behaviour of habituated animals.

The in-vitro and in-vivo data summarized above argue for a unique mechanism of action of OSU6162, ACR 16 and its congeners, with a stimulating component in lower concentrations/doses and an inhibitory component after higher concentrations/doses. It is suggested that the mechanism involved is more complicated than that of typical partial dopamine receptor agonists, in that not only the orthosteric but, in addition, an allosteric site on the dopamine D2 receptor takes part in the effect.

Finally, our studies revealed a previously unknown and surprising property of OSU6162, that is, a clearcut stimulating action in a classical animal Parkinson model (mice pretreated with reserpine and alpha-methyl-para-tyrosine).

Taken together, these data indicate a unique stabilizer profile of the phenylpiperidines of the present invention, that is, an activating effect in both moderate and severe dopaminergic hypofunction, showing up already in low dosage, and at the same time an ability to dampen a physiological or inappropriate degree of dopaminergic hyperactivity. Such a profile shows great promise in a variety of neurological and psychiatric disorders, in which a stabilization of dopamine function towards a normal level may prove beneficial.

### Features of the application

1. Use of a dopamine stabilizing substance selected from the group consisting of:
   compounds of formula I 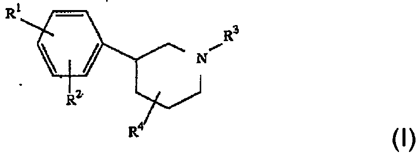 wherein:
      - R¹ and R²: are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, CO-COON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
      - R³: is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
      - R⁴: and R are independently selected from hydrogen, CF₃CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
      - R⁵: is phenyl, phenyl substituted with CN, CF3, CH₂CF₃, C₁ -C₈ alkyl, C₃-C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
      - R⁶ and R⁷: are independently H, C₁-C₈ alkyl, C₃-C₈ cycloalkyl, C₄-C₉ cycloalkyl-methyl, C₂-C₈ alkenyl or C₂-C₈ alkynyl;
   compounds of formula II 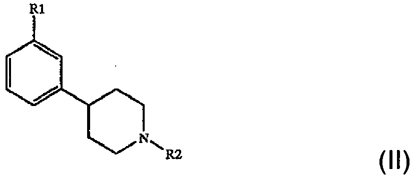 wherein:
      - R₁: is selected from the group consisting of OSO₂CF₃, OSO₂CH₃, SOR₃, SO₂R₃, COCH₃, and COCH₂CH₃, wherein R₃ is as defined below;
      - R₂: is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
      - R₃: is selected from the group consisting of C₁-C₃ alkyls, CF₃, and N(CH₃)₂;.
      wherein the compound of formula II does not have a high binding affinity
      to sigma receptors; and
   compounds of formula III 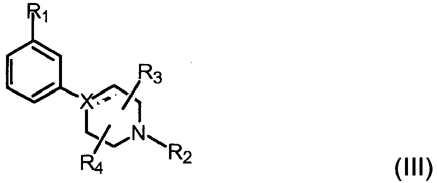 wherein:
      - X: is selected from the group consisting of N, CH, and C, however X may only be C when the compound comprises a double bond at the dotted line;
      - R₁: is selected from the group consisting of OSO₂CF₃, OSO₂CH₃, SOR₅, SO₂R₅, COR₅, CN, NO₂, CONHR₅, CF₃, 3-thiophene, 2-thiophene, 3-furane, 2-furane, F, Cl, Br, and I, wherein R₅ is as defined below;
      - R₂: is selected from the group consisting of C₁-C₄ alkyls, allyls, CH₂SCH₃, CH₂CH₂OCH₃, CH₂CH₂CH₂F, CH₂CF₃, 3,3,3-trifluoropropyl, 4,4,4-trifluorobutyl, and -(CH₂)-R₆, wherein R₆ is as defined below;
      - R₃ and R₄: are independently selected from the group consisting of H and C₁-C₄ alkyls, however both R₃ and R₄ cannot be H at the same time;
      - R₅: is selected from the group consisting of C₁-C₃ alkyls, CF₃, and N(R₂)₂, wherein R₂ is as defined above; and

      - R₆: is selected from the group consisting of C₃-C₆ cycloalkyls, 2-tetrahydrofurane, and 3-tetra-hydrofurane
   and pharmaceutically acceptable salts thereof
   for the manufacture of a medicament or a pharmaceutical composition for treatment of a neurological or psychiatric disorder characterized by a hypofunction of the dopamine system, wherein said substance is to be administered in an oral, subcutaneous or intramuscular daily dose of 1-20 mg or in an intravenous daily dose of 0.1-2 mg.
2. Use according to feature 1, wherein said neurological or psychiatric disorder characterized by a hypofunction of the dopamine system is selected from the group consisting of Parkinson's disease in early stages; restless legs; akathisia; dystonias; mental fatigue associated with high age, stroke, postencephalitic or posttraumatic conditions; attention-deficit disorders (ADHD); autism spectrum disorders; lapses of consciousness including narcolepsy, petit mal epilepsy and syncope; sleeping disorders including hypersomnia, sleep apnea, and attacks of sleep induced by dopamine receptor agonists; emesis and nausea; dopamine hypofuction induced by antipsychotic drugs.
3. Use according to feature 1 or 2, wherein the oral, subcutaneous or intramuscular daily dose is 2.5 to 15 mg.
4. Use of a dopamine stabilizing substance selected from the group consisting of:
   compounds of formula I 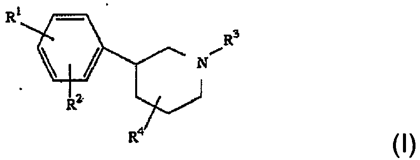 wherein:
      - R¹ and R²: are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, CO-COON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
      - R³: is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
      - R⁴ and R: are independently selected from hydrogen, CF₃CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
      - R⁵: is phenyl, phenyl substituted with CN, CF₃, CH₂CF₃, C₁ -C₈ alkyl, C₃-C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
      - R⁶ and R⁷: are independently H, C₁-C₈ alkyl, C₃-C₈ cycloalkyl, C₄-C₉ cycloalkyl-methyl, C₂-C₈ alkenyl or C₂-C₈ alkynyl;
   compounds of formula II 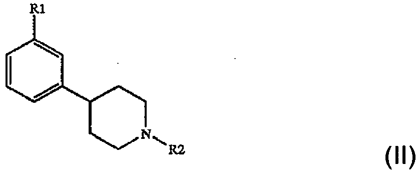 wherein:
      - R₁: is selected from the group consisting of OSO₂CF₃, OSO₂CH₃, SOR₃, SO₂R₃, COCH₃, and COCH₂CH₃, wherein R₃ is as defined below;
      - R₂: is selected from the group consisting of C₂-C₄ branched or unbranched alkyls, terminal allyl, CH₂CH₂OCH₃, CH₂CH₂CH₂F, CH₂CF₃, 3,3,3-trifluoropropyl, and 4,4,4-trifluorobutyl,
      - R₃: is selected from the group consisting of C₁-C₃ alkyls, CF₃, and N(CH₃)₂;.
      wherein the compound of formula II does not have a high binding affinity to sigma receptors; and
   compounds of formula III 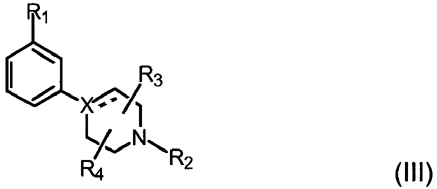 wherein:
      - X: is selected from the group consisting of N, CH, and C, however X may only be C when the compound comprises a double bond at the dotted line;
      - R₁: is selected from the group consisting of OSO₂CF₃, OSO₂CH₃, SOR₅, SO₂R₅, COR₅, CN, NO₂, CONHR₅, CF₃, 3-thiophene, 2-thiophene, 3-furane, 2-furane, F, Cl, Br, and I, wherein R₅ is as defined below;
      - R₂: is selected from the group consisting of C₁-C₄ alkyls, allyls, CH₂SCH₃, CH₂CH₂OCH₃, CH₂CH₂CH₂F, CH₂CF₃, 3,3,3-trifluoropropyl, 4,4,4-trifluorobutyl, and -(CH₂)-R₆, wherein R₆ is as defined below;
      - R₃ and R₄: are independently selected from the group consisting of H and C₁-C₄ alkyls, however both R₃ and R₄ cannot be H at the same time;
      - R₅: is selected from the group consisting of C₁-C₃ alkyls, CF₃, and N(R₂)₂, wherein R₂ is as defined above; and
      - R₆: is selected from the group consisting of C₃-C₆ cycloalkyls, 2-tetrahydrofurane, and 3-tetra-hydrofurane
   and pharmaceutically acceptable salts thereof
   for the manufacture of a medicament or a pharmaceutical composition for treatment of a disorder caused by instability of neural circuits, wherein said substance is to be administered in an oral, subcutaneous or intramuscular daily dose of 25-50 mg.
5. Use according to feature 4, wherein said disorder caused by instability of neural circuits is selected from the group consisting of schizophrenia; other psychoses and paranoid conditions; manic-depressive disorder; Huntington's disease; Tics; Tourette's disease; hiccup; dyskinesias induced by L-dopa and other dopamine-receptor agonists; tardive dyskinesias induced by long-term treatment with dopamine receptor antagonists; drug abuse and addiction; addiction to gambling; addiction to certain foodstuffs etc; emotional disorders including aggressiveness and pathological impulsiveness; emotional disturbances induced by severe pain; anxiety disorders including panic disorders, generalized anxiety disorder and obsessive-compulsive disorder; and adverse effects, including extrapyramidal, executive, cognitive and emotional caused by antipsychotic drugs.
6. Use according to any one of the preceding features, wherein a compound of formula I or a pharmaceutically acceptable salt thereof is used in the form of a pure enantiomer.
7. Use according to any one of the preceding features, wherein a compound of formula I or a pharmaceutically acceptable salt thereof is used, wherein R¹ is CN, OSOCF₃, or SO₂CH₃.
8. Use according to any one of the preceding features, wherein R² is H and R³ is C₁₋₈ alkyl.
9. Use according to any one of the preceding features, wherein R² is H and R³ is n-propyl.
10. Use according to any one of the preceding features, wherein R⁴ is H.
11. Use according to any one of the preceding features, wherein a compound of formula I or a pharmaceutically acceptable salt thereof is used, wherein R¹ is 3-OH, R² is H, R³ is n-propyl and R⁴ is C₂₋₈ alkyl.
12. Use according to any one of the preceding features, wherein said substance is S-(-)-3-[3-methylsulfonylphenyl]-1-propylpiperidine).
13. Use according to any one of features 1-5, wherein a compound of formula II or a pharmaceutically acceptable salt thereof is used, wherein R₁ is selected from the group consisting of OSO₂CF₃, OSO₂CH₃, SO₂CH₃, SO₂CF₃, COCH₃, and SO₂N(CH₃)₂.
14. Use according to feature 13, wherein R₁ is selected from the group consisting of SO₂CF₃, SO₂CH₃, and COCH₃.
15. Use according to any one of features 1-5, 13 or 14, wherein a compound of formula II or a pharmaceutically acceptable salt thereof is used, wherein R₂ is selected from the group consisting of n-propyl and ethyl.
16. Use according to any one of features 1-5 or 13-15, wherein a compound of formula II or a pharmaceutically acceptable salt thereof is used and said compound is 4-(3-methanesulfonylphenyl)-1-propyl-piperidine.
17. Use according to any one of features 1-5, wherein a compound of formula III or a pharmaceutically acceptable salt thereof is used, wherein X is CH or C.
18. Use according to feature any one of features 1-5 or 17, wherein a compound of formula III or a pharmaceutically acceptable salt thereof is used wherein R₁ is selected from the group consisting of OSO₂CF₃, OSO₂CH₃, SO₂Me, SO₂CF₃, COCH₃, CN, CF₃, CON(CH₃)₂ and SO₂N(CH₃)₂.
19. Use according to any one of features 1-5, 17 or 18, wherein a compound of formula III or a pharmaceutically acceptable salt thereof is used, wherein R₃ and R₄ both are CH₃.
20. Use according to any one of features 1-5 or 17-19, wherein a compound of formula III or a pharmaceutically acceptable salt thereof is used wherein R₁ is selected from the group consisting of SO₂CF₃, SO₂CH₃, COCH₃, CF₃, and CN, and X is CH.
21. Use according to any one of features 1-5 or 17-20, wherein a compound of formula III or a pharmaceutically acceptable salt thereof is used wherein R₂ is selected from the group consisting of n-propyl and ethyl.
22. Use according to any one of the preceding features, wherein the daily does is given as one dose a day.
23. Use according to any one of features 1-21, wherein the daily does is divided into two equal doses.

### References

Brandt-Christensen M, Andersen MB, Fink-Jensen A, Werge T, Gerlach J (2006) The substituted (S)-3-phenylpiperidine (-)-OSU6162 reduces apomorphine- and amphetamine-induced behaviour in Cebus apella monkeys. J Neural Transm 113(1): 11-19
Buxton ILO (2005) Pharmacokinetics and pharmacodynamics: the dynamics of drug absorption, distribution, action, and elimination. In: Brunton LL, Lazo JS, Parker KL (eds) Goodman & Gilman's the pharmacological basis of therapeutics. McGraw-Hill, New York, pp 1-39
Carlsson A (1983) Dopamine receptor agonists: intrinsic activity vs. state of receptor. J Neural Transm 57(4): 309-315
Carlsson A (2001) A half-century of neurotransmitter research: impact on neurology and psychiatry (Nobel lecture). Chembiochem 2(7-8): 484-493
Carlsson ML, Carlsson A, Nilsson M (2004) Schizophrenia: from dopamine to glutamate and back. Curr Med Chem 11 (3): 267-277
Carlsson A, Carlsson ML (2006) A dopaminergic deficit hypothesis of schizophrenia: the path to discovery. Dialogues Clin Neurosci 8(1):137-42
Centonze D, Usiello A, Gubellini P, Pisani A, Borrelli E, Bernardi G, Calabresi P (2002) Dopamine D2 receptor-mediated inhibition of dopaminergic neurons in mice lacking D2, receptors. Neuropsychopharmacology Nov;27(5):723-6
De Muth JE (1999) Basic statistics and pharmaceutical statistical applications, 1 st Marcel Dekker, Inc., New York, Pages
Gefvert O, Lindström LH, Dahlbäck O, Sonesson C, Waters N, Carlsson A, Tedroff J (2000) (-)-OSU6162 induces a rapid onset of antipsychotic effect after a single dose. A double-blind placebo-controlled pilot study. In: von Knorring L (ed) Scandinavian Society for Psychopharmacology, 41 st annual meeting. Nord J Psychiat, Copenhagen, Denmark, p 93-94
Grubbs FE (1969) Procedures for detecting outlying observations in samples. Technometrics 11 (1): 1-21
Hartter S, Huwel S, Lohmann T, Abou El Ela A, Langguth P, Hiemke C, Galla HJ (2003) How does the benzamide antipsychotic amisulpride get into the brain?--An in vitro approach comparing amisulpride with clozapine. Neuropsychopharmacology 28(11): 1916-1922
Jordan S, Chen R, Johnson J, Regardie K, Tadori Y, Kikuchi T (2002a) Aripiprazole is a potent, partial agonist at cloned human D2L and native rat 5-HT1A receptors. European Neuropsychopharmacology 12(Suppl. 3): 293-294
Jordan S, Johnson JL, Regardie K, Chen R, Koprivica V, Tadori Y, Kambayashi J, Kitagawa H, Kikuchi T (2007) Dopamine D2 receptor partial agonists display differential or contrasting characteristics in membrane and cell-based assays of dopamine D2 receptor signaling. Prog Neuropsychopharmacol Biol Psychiatry 31(2): 348-356
Jordan S, Koprivica V, Chen R, Tottori K, Kikuchi T, Altar CA (2002b) The antipsychotic aripiprazole is a potent, partial agonist at the human 5-HT1A receptor. Eur J Pharmacol 441(3): 137-140
Jordan S, Regardie K, Johnson JL, Chen R, Kambayashi J, McQuade R, Kitagawa H, Tadori Y, Kikuchi T (2006) In vitro functional characteristics of dopamine D2 receptor partial agonists in second and third messenger-based assays of cloned human dopamine D2Long receptor signalling. J Psycho-pharmacol
Khan ZU, Mrzljak L, Gutierrez A, de la Calle A, Goldman-Rakic PS (1998) Prominence of the dopamine D2 short isoform in dopaminergic pathways. Proc Natl Acad Sci USA. Jun 23;95(13):7731-6
Lahti RA, Figur LM, Piercey MF, Ruppel PL, Evans DL (1992) Intrinsic activity determinations at the dopamine D2 guanine nucleotide-binding protein-coupled receptor: utilization of receptor state binding affinities. Mol Pharmacol Sep;42(3):432-8
Lahti RA, Tamminga CA, Carlsson A (2007) Stimulating and inhibitory effects of the dopamine "stabilizer" (-)-OSU6162 on dopamine D(2) receptor function in vitro. J Neural Transm
Leysen JE, Gommeren W, Mertens J, Luyten WH, Pauwels PJ, Ewert M, Seeburg P (1993) Comparison of in vitro binding properties of a series of dopamine antagonists and agonists for cloned human dopamine D2S and D2L receptors and for D2 receptors in rat striatal and mesolimbic tissues, using [125l] 2'-iodospiperone. Psychopharmacology (Berl) 110(1-2):27-36
Lundberg T, Tedroff J, Waters N, Sonesson C, Carlsson A, Hagström P, Lindström LH, Gefvert O (2002) Safety of early clinical experience with (-)-OSU6162, a dopaminergic stabilizer with antipsychotic properties
Malmberg A, Jackson DM, Eriksson A, Mohell N (1993) Unique binding characteristics of antipsychotic agents interacting with human dopamine D2A, D2B, and D3 receptors. Mol Pharmacol May;43(5):749-54
Malmberg, Mikaels, Mohell N (1998) Agonist and inverse agonist activity at the dopamine D3 receptor measured by guanosine 5'--gamma-thio-triphosphate--35S- binding. J Pharmacol Exp Ther 285(1): 119-126
Natesan S, Svensson KA, Reckless GE, Nobrega JN, Barlow KB, Johansson AM, Kapur S (2006) The dopamine stabilizers (S)-(-)-(3-methanesulfonylphenyl)-1-propyl-piperidine [(-)-OSU6162] and 4-(3-methanesulfonylphenyl)-1-propyl-piperidine (ACR16) show high in vivo D2 receptor occupancy, antipsychotic-like efficacy, and low potential for motor side effects in the rat. J Pharmacol Exp Ther 318(2): 810-818
Neu H, Hartvig P, Torstenson R, Fasth KJ, Sonesson C, Waters N, Carlsson A, Tedroff J, Langstrom B (1997) Synthesis of [11C-methyl]-(-)-OSU6162, its regional brain distribution and some pharmacological effects of (-)-OSU6162 on the dopaminergic system studied in the rhesus monkey by positron emission tomography. Nucl Med Biol Aug;24(6):507-11
Perrault G, Depoortere R, Morel E, Sanger DJ, Scatton B (1997) Psychopharmacological profile of amisulpride: an antipsychotic drug with presynaptic D2/D3 dopamine receptor antagonist activity and limbic selectivity. J Pharmacol Exp Ther 280(1): 73-82
Pontén H, Carlsson A, Kullingsjö J, Sonesson C, Waters ES, Waters N, Tedroff J (2002) The dopamine stabilizer ACR16 prevents L-DOPA induced sensitization in the 6-OHDA-lesioned rat. 32nd Annual Meeting, Society for neuroscience. Soc Neurosci, Orlando Program No. 787.6
Rebec GV (1998) Real-time assessments of dopamine function during behavior: single-unit recording, iontophoresis, and fast-scan cyclic voltammetry in awake, unrestrained rats. Alcohol Clin Exp Res 22(1): 32-40
Rung JP, Carlsson A, Rydén Markinhuhta K, Carlsson ML (2005a) The dopaminergic stabilizers (-)-OSU6162 and ACR16 reverse (+)-MK-801-induced social withdrawal in rats. Prog Neuropsychopharmacol Biol Psychiatry 29(5): 833-839
Rung JP, Carlsson A, Rydén Markinhuhta K, Carlsson ML (2005b) (+)-MK-801 induced social withdrawal in rats; a model for negative symptoms of schizophrenia. Prog Neuropsychopharmacol Biol Psychiatry 29(5): 827-832 Schoemaker H, Claustre Y, Fage D, Rouquier L, Chergui K, Curet O, Oblin A, Gonon F, Carter C, Benavides J, Scatton B (1997) Neurochemical characteristics of amisulpride, an atypical dopamine D2/D3 receptor antagonist with both presynaptic and limbic selectivity. J Pharmacol Exp Ther 280(1): 83-97
Seeman P, Guan HC (2006) Dopamine partial agonist action of (-)OSU6162 is consistent with dopamine hyperactivity in psychosis. Eur J Pharmacol
Siegel S, Castellan NJ (1988) The Kruskal-Wallis one-way analysis of variance by ranks Nonparametric statistics for the behavioral sciences. McGraw-Hill, New York, pp 206-216
Sokoloff P, Griffon N, Gullin O, Diaz J, Pilon C, Schwartz J-C (2003) The dopamine D3 receptor in the pathophysiology and treatment of neuropsychiatric disorders. In: Sidhu A, Laruelle M, Vernier P (eds) Dopamine receptors and transporters. Marcel Dekker, New York, pp 211-253
Sonesson C, Lin CH, Hansson L, Waters N, Svensson K, Carlsson A, Smith MW, Wikstrom H (1994) Substituted (S)-phenylpiperidines and rigid congeners as preferential dopamine autoreceptor antagonists: synthesis and structure-activity relationships. J Med Chem 37(17): 2735-2753
Ståhle L, Ungerstedt U (1986) Effects of neuroleptic drugs on the inhibition of exploratory behaviour induced by a low dose of apomorphine: implications for the identity of dopamine receptors. Pharmacol Biochem Behav 25(2): 473-480
Svensson K, Johansson AM, Magnusson T, Carlsson A (1986) (+)-AJ 76 and (+)-UH 232: central stimulants acting as preferential dopamine autoreceptor antagonists. Naunyn Schmiedebergs Arch Pharmacol 334(3): 234-245
Tadori Y, Kitagawa H, Forbes RA, McQuade RD, Stark A, Kikuchi T (2007) Differences in agonist/antagonist properties at human dopamine D(2) receptors between aripiprazole, bifeprunox and SDZ 208-912. Eur J Pharmacol Tadori Y, Miwa T, Tottori K, Burris KD, Stark A, Mori T, Kikuchi T (2005) Aripiprazole's low intrinsic activities at human dopamine D2L and D2S receptors render it a unique antipsychotic. Eur J Pharmacol 515(1-3): 10-19
Tedroff J, Ekesbo A, Sonesson C, Waters N, Carlsson A (1999) Long-lasting improvement following (-)-OSU6162 in a patient with Huntington's disease. Neurology 53(7): 1605-1606
Waters N, Carlsson A, Carlsson M, Martin P, Tedroff J, Kullingsjö J, Pettersson F, Sonesson C, Waters ES (2002) Pharmacology of ACR16, a dopamine stabilizer. 32nd Annual Meeting, Society for neuroscience. Soc Neurosci, Orlando Program No. 894.13
Waters N, Lofberg L, Haadsma Svensson S, Svensson K, Sonesson C, Carlsson A (1994) Differential effects of dopamine D2 and D3 receptor antagonists in regard to dopamine release, in vivo receptor displacement and behaviour. J Neural Transm Gen Sect 98(1): 39-55
Waters N, Svensson K, Haadsma Svensson SR, Smith MW, Carlsson A (1993) The dopamine D3-receptor: a postsynaptic receptor inhibitory on rat locomotor activity. J Neural Transm Gen Sect 94(1): 11-19

## Claims

1. Use of S-(-)-3-[3-methylsulfonylphenyl]-1-propylpiperidine
or a pharmaceutically acceptable salt thereof
for the manufacture of a medicament or a pharmaceutical composition for treatment of Parkinson's disease in early stages or for treatment of mental fatigue associated with high age, stroke, postencephalitic or posttraumatic conditions, wherein S-(-)-3-[3-methylsulfonylphenyl]-1-propylpiperidine is to be administered in an oral, subcutaneous or intramuscular daily dose of 1-20 mg or in an intravenous daily dose of 0.1-2 mg.

2. Use according to claim 1, wherein said medicament or pharmaceutical composition is for treatment of Parkinson's disease in early stages.

3. Use according to claim 1, wherein said medicament or pharmaceutical composition is for treatment of mental fatigue associated with high age, stroke, postencephalitic or posttraumatic conditions.

4. Use according to any one of the claims 1-3, wherein the oral, subcutaneous or intramuscular daily dose is 2.5 to 15 mg.

5. Use according to any one of claims 1-4, wherein the daily does is given as one dose a day.

6. Use according to any one of claim 1-4, wherein the daily does is given in two equal doses.

7. S-(-)-3-[3-methylsulfonylphenyl]-1-propylpiperidine or a pharmaceutically acceptable salt thereof for use in the treatment of Parkinson's disease in early stages or in the treatment of mental fatigue associated with high age, stroke, postencephalitic or posttraumatic conditions, wherein S-(-)-3-[3-methylsulfonylphenyl]-1-propylpiperidine is to be administered in an oral, subcutaneous or intramuscular daily dose of 1-20 mg or in an intravenous daily dose of 0.1-2 mg.

8. S-(-)-3-[3-methylsulfonylphenyl]-1-propylpiperidine or a pharmaceutically acceptable salt thereof for use according to claim 7 for treatment of Parkinson's disease in early stages.

9. S-(-)-3-[3-methylsulfonylphenyl]-1-propylpiperidine or a pharmaceutically acceptable salt thereof for use according to claim 7 for treatment of mental fatigue associated with high age, stroke, postencephalitic or posttraumatic conditions

10. S-(-)-3-[3-methylsulfonylphenyl]-1-propylpiperidine or a pharmaceutically acceptable salt thereof for use according to any one of the claims 1-9, wherein the oral, subcutaneous or intramuscular daily dose is 2.5 to 15 mg.

11. S-(-)-3-[3-methylsulfonylphenyl]-1-propylpiperidine or a pharmaceutically acceptable salt thereof for use according to any one of the claims 7-11, wherein the daily does is given as one dose a day.

12. S-(-)-3-[3-methylsulfonylphenyl]-1-propylpiperidine or a pharmaceutically acceptable salt thereof for use according to any one of the claims 7-11, wherein the daily does is given in two equal doses.

## Patentansprüche

1. Verwendung von S-(-)-3-[3-Methylsulfonylphenyl]-1-propylpiperidin oder einem pharmazeutisch verträglichen Salz davon
für die Herstellung eines Arzneimittels oder einer pharmazeutischen Zusammensetzung zur Behandlung der Parkinson-Krankheit im Frühstadium oder zur Behandlung von geistiger Erschöpfung in Verbindung mit hohem Alter, Schlaganfall, postenzephalitischen oder posttraumatischen Zuständen, wobei S-(-)-3-[3-Methylsulfonylphenyl]-1-propylpiperidin in einer täglichen Dosis von 1-20 mg oral, subkutan oder intramuskulär oder in einer täglichen Dosis von 0,1-2 mg intravenös zu verabreichen ist.

2. Verwendung gemäß Anspruch 1, wobei das Arzneimittel bzw. die pharmazeutische Zusammensetzung zur Behandlung der Parkinson-Krankheit im Frühstadium vorgesehen ist.

3. Verwendung gemäß Anspruch 1, wobei das Arzneimittel bzw. die pharmazeutische Zusammensetzung zur Behandlung von geistiger Erschöpfung in Verbindung mit hohem Alter, Schlaganfall, postenzephalitischen oder posttraumatischen Zuständen vorgesehen ist.

4. Verwendung gemäß einem der Ansprüche 1-3, wobei die tägliche orale, subkutane oder intramuskuläre Dosis 2,5 bis 15 mg beträgt.

5. Verwendung gemäß einem der Ansprüche 1-4, wobei die tägliche Dosis als Einzeldosis pro Tag verabreicht wird.

6. Verwendung gemäß einem der Ansprüche 1-4, wobei die tägliche Dosis in zwei gleichen Gaben verabreicht wird.

7. S-(-)-3-[3-Methylsulfonylphenyl]-1-propylpiperidin oder ein pharmazeutisch verträgliches Salz davon zur Verwendung bei der Behandlung der Parkinson-Krankheit im Frühstadium oder bei der Behandlung von geistiger Erschöpfung in Verbindung mit hohem Alter, Schlaganfall, postenzephalitischen oder posttraumatischen Zuständen, wobei S-(-)-3-[3-Methylsulfonylphenyl]-1-propylpiperidin in einer täglichen Dosis von 1-20 mg oral, subkutan oder intramuskulär oder in einer täglichen Dosis von 0,1-2 mg intravenös zu verabreichen ist.

8. S-(-)-3-[3-Methylsulfonylphenyl]-1-propylpiperidin oder ein pharmazeutisch verträgliches Salz davon zur Verwendung gemäß Anspruch 7 zur Behandlung der Parkinson-Krankheit im Frühstadium.

9. S-(-)-3-[3-Methylsulfonylphenyl]-1-propylpiperidin oder ein pharmazeutisch verträgliches Salz davon zur Verwendung gemäß Anspruch 7 zur Behandlung von geistiger Erschöpfung in Verbindung mit hohem Alter, Schlaganfall, postenzephalitischen oder posttraumatischen Zuständen.

10. S-(-)-3-[3-Methylsulfonylphenyl]-1-propylpiperidin oder ein pharmazeutisch verträgliches Salz davon zur Verwendung gemäß einem der Ansprüche 1-9, wobei die tägliche orale, subkutane oder intramuskuläre Dosis 2,5 bis 15 mg beträgt.

11. S-(-)-3-[3-Methylsulfonylphenyl]-1-propylpiperidin oder ein pharmazeutisch verträgliches Salz davon zur Verwendung gemäß einem der Ansprüche 7-11, wobei die tägliche Dosis als Einzeldosis pro Tag verabreicht wird.

12. S-(-)-3-[3-Methylsulfonylphenyl]-1-propylpiperidin oder ein pharmazeutisch verträgliches Salz davon zur Verwendung gemäß einem der Ansprüche 7-11, wobei die tägliche Dosis in zwei gleichen Gaben verabreicht wird.

## Revendications

1. Emploi de S-(-)-3-[3-méthylsulfonylphényl]-1-propylpipéridine ou l'un de ses sels de qualité pharmaceutique
dans la fabrication d'un médicament ou d'une composition pharmaceutique destinés au traitement de la maladie de Parkinson aux stades précoces ou au traitement de la fatigue mentale associée à un âge important, un accident vasculaire cérébral, des états pathologiques post-encéphalitiques ou post-traumatiques, la S-(-)-3-[3-méthylsulfonylphényl]-1-propylpipéridine devant être administrée en une dose journalière orale, sous-cutanée ou intramusculaire de 1 à 20 mg ou en une dose journalière intraveineuse de 0,1 à 2 mg.

2. Emploi conforme à la revendication 1, où ledit médicament ou ladite composition pharmaceutique sont destinés au traitement de la maladie de Parkinson aux stades précoces.

3. Emploi conforme à la revendication 1, où ledit médicament ou ladite composition pharmaceutique sont destinés au traitement de la fatigue mentale associée à un âge important, un accident vasculaire cérébral, des états pathologiques post-encéphalitiques ou post-traumatiques.

4. Emploi conforme à l'une quelconque des revendications 1 à 3, où la dose journalière orale, sous-cutanée ou intramusculaire est comprise entre 2,5 et 15 mg.

5. Emploi conforme à l'une quelconque des revendications 1 à 4, où la dose journalière est administrée sous forme d'une dose unique par jour.

6. Emploi conforme à l'une quelconque des revendications 1 à 4, où la dose journalière est administrée sous forme de deux doses égales.

7. S-(-)-3-[3-méthylsulfonylphényl]-1-propylpipéridine ou l'un de ses sels de qualité pharmaceutique, pour emploi dans le traitement de la maladie de Parkinson aux stades précoces ou dans le traitement de la fatigue mentale associée à un âge important, un accident vasculaire cérébral, des états pathologiques post-encéphalitiques ou post-traumatiques, la S-(-)-3-[3-méthylsulfonylphényl]-1-propylpipéridine devant être administrée à une dose journalière orale, sous-cutanée ou intramusculaire de 1 à 20 mg ou à une dose journalière intraveineuse de 0,1 à 2 mg.

8. S-(-)-3-[3-méthylsulfonylphényl]-1-propylpipéridine ou l'un de ses sels de qualité pharmaceutique pour emploi conforme à la revendication 7 dans le traitement de la maladie de Parkinson aux stades précoces.

9. S-(-)-3-[3-méthylsulfonylphényl]-1-propylpipéridine ou l'un de ses sels de qualité pharmaceutique pour emploi conforme à la revendication 7 dans le traitement de la fatigue mentale associée à un âge important, un accident vasculaire cérébral, des états pathologiques post-encéphalitiques ou post-traumatiques.

10. S-(-)-3-[3-méthylsulfonylphényl]-1-propylpipéridine ou l'un de ses sels de qualité pharmaceutique pour emploi conforme à l'une quelconque des revendications 1 à 9, où la dose journalière orale, sous-cutanée ou intramusculaire est comprise entre 2,5 et 15 mg.

11. S-(-)-3-[3-méthylsulfonylphényl]-1-propylpipéridine ou l'un de ses sels de qualité pharmaceutique pour emploi conforme à l'une quelconque des revendications 7 à 11, où la dose journalière est administrée sous forme d'une dose unique par jour.

12. S-(-)-3-[3-méthylsulfonylphényl]-1-propylpipéridine ou l'un de ses sels de qualité pharmaceutique pour emploi conforme à l'une quelconque des revendications 7 à 11, où la dose journalière est administrée sous forme de deux doses égales.
